# EUROPÄISCHE PATENTSCHRIFT

(11) **EP 1 884 507 B1**
(45) Veröffentlichungstag und Bekanntmachung des Hinweises auf die Patenterteilung: **12.05.2010**
(21) Anmeldenummer: 07014149.4
(22) Anmeldetag: 19.07.2007
(51) Int. Cl.: C07C 27/02, C07C 29/09, C07C 29/128, C07C 29/74, C07C 31/22, A61K 47/10

(54) **Verfahren zur Herstellung von Glycerin**
Method for manufacturing glycerine
Procédé destiné à la fabrication de glycérine

(30) Priorität: 27.07.2006 DE 102006034702
(43) Veröffentlichungstag der Anmeldung: 06.02.2008
(73) Patentinhaber: Cognis IP Management GmbH, 40589 Düsseldorf (DE)
(72) Erfinder: Gutsche, Bernhard, 40724 Hilden (DE); Strube, Albert, 41470 Neuss (DE); Meyer, Carolin, 40589 Düsseldorf (DE); Rigal, Jean, 31170 Tournefeuille (FR); Eychenne, Valerie, 31400 Toulouse (FR); Richard-Elsner, Christiane, 40595 Düsseldorf (DE); Josten, Horst, 40593 Düsseldorf (DE); Schörken, Ulrich, 40591 Düsseldorf (DE); Melchior, David, 51465 Bergisch Gladbach (DE); Seifert, Jochen, 64367 Mühltal (DE)

(56) Entgegenhaltungen:
- EP-A- 1 092 703
- EP-A- 1 242 121
- US-A- 2 505 735
- LAGO R C A ET AL: "EXTRACTION AND TRANSESTERIFICATION OF VEGETABLE OILS WITH ETHANOL" OLEAGINEUX, PARIS, FR, Bd. 40, Nr. 3, 1985, Seiten 147-154, XP001248887 ISSN: 0030-2082

## Beschreibung

Die vorliegende Erfindung betrifft ein Verfahren zur Herstellung von Glycerin.

Glycerin ist unter anderem als Rohstoff für Arzneimittel von Bedeutung. Glycerin kann aus pflanzlichen Ölen oder Fetten, aus tierischen Ölen oder Fetten und synthetisch ausgehend von Propylen hergestellt werden.

Für pharmazeutische Anwendungen, die eine hohe Reinheit des Glycerins erfordern, wird zumeist synthetisch hergestelltes Glycerin eingesetzt, das aus Propylen gewonnen wurde. Glycerin basierend auf pflanzlichen oder tierischen Fetten besitzt zumeist nicht die geforderte Qualität.

Pflanzliches Glycerin kann aus verschiedenen Quellen gewonnen werden, zum Beispiel aus Pulp-Ölen wie zum Beispiel Palm- und Olivenöl oder aus Samen-Ölen wie zum Beispiel Sojaöl, Rapsöl, Sonnenblumenöl, Distelöl, Leinöl, Erdnussöl, Kokosöl oder Palmkernöl.

Es existieren verschiedene Prozesse, die pflanzliche oder tierische Fette oder Öle in Glycerin und Fettsäuren oder Ester von Fettsäuren umsetzen. Gängige Methoden sind zum Beispiel die Hochdruckspaltung von Ölen, die Hochtemperaturumesterung mit Methanol in Anwesenheit von Zinkkatalysatoren oder die alkalische Niederdruckumesterung mit Methanol. Alternative neuere Verfahren, die bisher kaum industriell eingesetzt werden, sind zum Beispiel die enzymatische Hydrolyse oder die Umesterung von pflanzlichen Ölen sowie die Niedertemperaturumesterung mit Phasentransferkatalysatoren. Im Zusammenhang mit diesen Prozessen wird üblicherweise von einer Ölphase und von einer Glycerinphase gesprochen. Die Ölphase ist entweder die Fettsäure-Phase erhältlich durch Ölspaltung oder die Methylesterphase erhältlich durch Umesterung; Die Glycerinphase setzt sich unterhalb der Ölphase ab und kann z.B. durch Dekantieren abgetrennt werden.

Glycerin kann zur weiteren Aufreinigung von der Ölphase abgetrennt und dann über verschiedene Methoden weiter gereinigt werden. Zur Abtrennung der Ölphase werden zumeist Abscheider benutzt, die Entfernung von Ölresten (Ölreste bedeutet entweder Fettsäure oder Methylester sowie einige (Partial)glyceride aus unvollständiger Umsetzung des Öls) kann dann über pH-Shift und Absetzbecken durchgeführt werden. Methoden zur weiteren Reinigung, die industriell genutzt werden, sind zum Beispiel die fraktionierte Destillation, chromatographische Methoden wie Ionenausschlußchromatographie zur Entsalzung oder die Benutzung von Harzen wie Ionentauschern oder Aminharzen zur selektiven Bindung von Nebenprodukten. Als weiteres können absorptive Methoden wie zum Beispiel Aktivkohle- oder Bleicherde-Behandlungen durchgeführt werden, um Farb- und Geruchsstoffe zu beseitigen.

Je nach eingesetztem Pflanzenöl oder Pflanzenfett oder tierischem Öl oder Fett, je nach industriellem Prozess der Ölverarbeitung und nach Kombination der Glycerinaufarbeitung wird Glycerin unterschiedlicher Qualität erzeugt (siehe hierzu auch die Beispiele der vorliegenden Schrift). Das nach Standardverfahren aufgearbeitete Glycerin weist in Lagertests keine gute Oxidationsstabilität auf und es wird eine deutliche Bildung von Aldehyden und Ketonen detektiert.

Glycerin kann als Verunreinigung Aldehyde und Ketone enthalten. Aldehyde und Ketone können, wenn sie mit Proteinen in Kontakt kommen, mit den Amingruppen von Proteinen Schiffsche Basen bilden und somit die Proteine modifizieren und zum Teil sogar vernetzen. Dies ist insbesondere negativ für den Einsatz von Glycerin als Rohstoff für Arzneimittel. Dies ist zum einen so, weil viele Arzneimittel Proteine enthalten. Zum anderen ist das so, weil die Arzneimittel, wenn sie mit dem menschlichen Körper in Kontakt kommen, mit Proteinen in Kontakt kommen.

Daher ist es wichtig, insbesondere für pharmazeutische Anwendungen, Glycerin herzustellen, das einen möglichst niedrigen Gehalt an Aldehyden und Ketonen hat. Weiterhin soll das Glycerin nach Möglichkeit keine Aldehyde oder Ketone (z. B. durch Oxidation) während seiner Lagerung bilden.

EP-B1 242 121 (Patentinhaber Eli Lilly) offenbart Glycerin mit einem niedrigen Gehalt an Aldehyden und pharmazeutische Zusammensetzungen enthaltend dieses Glycerin. Das Glycerin kann pflanzlichen Ursprungs sein oder aus Propylen synthetisch hergestellt worden sein. Weiterhin wird ein Verfahren zur Herstellung dieses Glycerins offenbart, wobei der Aldehydgehalt des Glycerins durch aminogruppenhaltige Polymerharze verringert wird.

US-A-2 505 735 offenbart, dass nach der Verseifung erhaltenes Rohglycerin mit einem Reduktionsmittel aus Zinkstaub und Säure versetzt wird.

Der vorliegenden Erfindung liegt die Aufgabe zu Grunde, ein Verfahren zur Herstellung von Glycerin bereit zu stellen, wobei das hergestellte Glycerin einen niedrigen Gehalt an Aldehyden und Ketonen haben soll und wobei das Glycerin lagerstabil sein soll. Lagerstabil bedeutet, dass bei der Lagerung des Glycerins nur wenig Aldehyde oder Ketone gebildet werden sollen.

Diese Aufgabe wird gelöst durch ein Verfahren zur Herstellung von Glycerin nach Anspruch 1 Bereitstellung eines pflanzlichen Öls oder Fetts, die Gewinnung von Roh-Glycerin aus dem pflanzlichen Öl oder Fett und die Behandlung des Roh-Glycerins mit einem Reduktionsmittel, bevorzugt mit einem Hydrierungsmittel, bevorzugt mit einem Borhydrid, insbesondere mit Natriumborhydrid. Dieses Verfahren ist Gegenstand der vorliegenden Erfindung.

Das erfindungsgemäße Glycerin unterscheidt sich vom Stand der Technik durch seine hohe Lagerstabilität, d. h. durch die geringe Bildung von Glycerinaldehyd und Dihydroxyaceton bei der Lagerung.

Das erfindungsgemäße Verfahren zur Herstellung von Glycerin hat zahlreiche Vorteile. Unter anderem kann es so geführt werden, dass das nach dem erfindungsgemäßen Verfahren hergestellte Glycerin die Anforderungen für pharmazeutische Rohstoffe wie definiert in dem in der Pharmabranche einschlägigen Standardwerk Pharmacopeia erfüllt.

Erfolgt bei dem erfindungsgemäße Verfahren zur Herstellung von Glycerin eine Reinigung des Roh-Glycerins durch Destillation des vor der Behandlung mit einem Reduktionsmittel, dann kann ein besonders reines und lagerstabiles Glycerin erhalten werden bei behandelt bei gleichzeitig geringem Bedarf an Reduktionsmittel.

Glycerin pflanzlichen Ursprungs erhältlich gemäß dem erfindungsgemäßen Verfahren ist lagerstabiler als Glycerin aus Propylen, das einem Reduktionsschritt unterzogen wurde. Dies zeigen insbesondere die Beispiele der vorliegenden Schrift.

Mit dem erfindungsgemäßen Verfahren kann Glycerin mit einem sehr niedrigen Gehalt an Aldehyden und Ketonen hergestellt werden. Es kann lagerstabiles Glycerin hergestellt werden. Lagerstabilität bedeutet insbesondere hohe Oxidationsstabilität während der Lagerung, d. h. dass keine signifikante Bildung von Aldehyden oder Ketonen während der Lagerung des Glycerins erfolgt. Als Maßstab kann hier die Lagerung über einen Zeitraum von 8 Wochen herangezogen werden.

Es kann vorteilhaft sein, in dem erfindungsgemäßen Verfahren geeignete Rohstoffe einzusetzen, insbesondere solche pflanzlichen Fette oder Öle, die dazu beitragen, dass der Gehalt an Aldehyden und Ketonen im erhaltenen Glycerin niedrig ist.

Es kann vorteilhaft sein, in dem erfindungsgemäßen Verfahren die Gewinnung von Roh-Glycerin aus dem pflanzlichen Fett oder Öl, die nach bekannten Verfahren durchgeführt werden kann, nach einem solchen Verfahren durchzuführen, das dazu beiträgt, dass der Gehalt an Aldehyden und Ketonen im erhaltenen Glycerin niedrig ist.

Es kann vorteilhaft sein, in dem Verfahren zur Gewinnung von Roh-Glycerin aus dem pflanzlichen Fett oder Öl an sich bekannte Aufarbeitungsschritte vorzusehen (zum Beispiel eine Destillation, insbesondere eine fraktionierte Destillation), die dazu beitragen, dass der Gehalt an Aldehyden und Ketonen im erhaltenen Glycerin niedrig ist.

Es kann vorteilhaft sein, in dem Verfahren zur Gewinnung von Roh-Glycerin aus dem pflanzlichen Fett oder Öl neben der Reduktion an sich bekannte, weitere Reinigungsschritte vorzusehen. Denkbar ist insbesondere eine Reinigung mit aminhaltigen Verbindungen, die chemisch mit verunreinigenden Aldehyden und Ketonen reagieren. Die aminhalatigen Verbindungen können aminofunktionalisierte Harze sein, es kann aber auch eine Umsetzung mit Formaldehyd oder Diaminen oder eine Reaktion mit Alkylaminen wie Steraylamin und dann eine Abtrennung des öllöslichen Amins über Extraktion erfolgen. Weiterhin möglich ist eine Adsorbtion, z.B. auf Adsorberharzen oder Aktivkohle. Auch eine katalytische Oxidation der Aldehyde zu Carbonsäuren und der Ketone zu Carbonsäuren unter C-Kettenspaltung sind möglich. Diese Verfahren tragen dazu bei, dass der Gehalt an Aldehyden und Ketonen im erhaltenen Glycerin niedrig ist.

Das Glycerin, das durch das erfindungsgemäße Verfahren hergestellt wird, eignet sich insbesondere für pharmazeutische Anwendungen, insbesondere für die Anwendung in Mischungen mit Proteinen oder anderen aminhaltigen Verbindungen, insbesondere auch zur parenteralen Anwendung.

Das erfindungsgemäße Verfahren geht aus von einem pflanzlichen Öl oder Fett. Dieses pflanzliche Öl oder Fett kann insbesondere Sonnenblumenöl oder Sojaöl oder "Rapsöl low erucic" sein. Letzteres ist Rapsöl aus einer Raps-Züchtungsvariante mit einem niedrigen Gehalt an Erucasäure (< 1 Gew.-%), das z. B. zur Ernährung verwendet wird. Das entsprechende High erucic Rapsöl geht üblicherweise in technische Anwendungen.

Die Gewinnung von Roh-Glycerin aus dem pflanzlichen Öl oder Fett kann nach an sich bekannten Verfahren erfolgen. Diese können insbesondere Niederdruckprozesse und Niedertemperaturprozesse sein. Insbesondere können herangezogen werden eine alkalische Niederdruckumesterung, eine Umesterung über Phasentransferkatalyse, eine Umesterung über enzymatische Katalyse oder eine enzymatische Hydrolyse. Bevorzugt sind sogenannte Niederdruck-Niedertemperaturverfahren, d. h. Verfahren, die ausschließlich bei Temperaturen von unter 100 °C und bei Drücken von unter 2 bar (Absolutdruck) betrieben werden. Insbesondere können hier genannt werden:
- die alkalische Niederdruckumesterung: Temperatur etwa 80 °C, Druck < 2 bar, Katalysator Natrium- oder Kaliummethylat, 2- oder 3-stufiges Verfahren mit Glycerinentfernung im Prozeß;
- die Phasentransferkatalyse: Typische Bedingungen: 60 °C, Normaldruck, Katalysator z.B. quartäre Aminverbindungen (z.B. Aliquat-Typen der Firma Cognis Deutschland GmbH & Co. KG, Düsseldorf, Deutschland);
- die enzymatische Katalyse: Typische Bedingungen: 30 - 60 °C, Normaldruck, Katalysator Lipase in freier oder immobilisierter Form.

Rohglycerin im Sinne der vorliegenden Erfindung ist jedes Glycerin vor seiner Behandlung mit einem Reduktionsmittel. Roh-Glycerin kann also Glycerin verschiedener Reinheit bezeichnen. Roh-Glycerin kann beispielsweise Glycerin sein, das unmittelbar, ohne weitere Reinigungsschritte aus der Fettspaltung erhalten wird. Roh-Glycerin kann Glycerin sein, das bereits Reinigungsschritten, wie z.B. einer Destillation, unterzogen wurde. Insbesondere kann Roh-Glycerin im Sinne der vorliegenden Erfindung Glycerin sein, das die Reinheitsanforderungen für pharmazeutische Anwendungen nach EP/USP erfüllt (so genanntes EP/USP pharma grade Glycerin).

Das erfindungsgemäße Verfahren zur Herstellung von Glycerin kann weitere Aufarbeitungsschritte oder Reinigungsschritte umfassen. Diese Schritte können entweder vor oder nach der Behandlung des Roh-Glycerins mit einem Reduktionsmittel vorgesehen werden. Insbesondere kann eine fraktionierte Destillation vorgesehen werden. Diese kann insbesondere vor der Behandlung des Roh-Glycerins mit einem Reduktionsmittel vorgesehen werden. Weiterhin kann eine Kombination aus Destillation, insbesondere fraktionierter Destillation, und einer Behandlung mit Aktivkohle vorgesehen werden. Diese Kombination kann insbesondere vor der Behandlung des Roh-Glycerins mit einem Reduktionsmittel vorgesehen werden.

Das erfindungsgemäße Verfahren zur Herstellung von Glycerin umfasst die Behandlung des Roh-Glycerins mit einem Reduktionsmittel. Als Reduktionsmittel kommen in Frage: Metallhydride wie z.B. LiAlH4, NaBH4 und andere, molekularer Wasserstoff unter Metallkatalyse mit z.B. Nickel und Platin; reduzierender anorganischer Stickstoff, Schwefel und Phosphorverbindungen wie z.B. hypophosphorige Säure. Als Reduktionsmittel kann insbesondere ein Hydrierungsmittel, insbesondere ein Borhydrid, insbesondere Natriumborhydrid vorgesehen werden. Das Borhydrid kann insbesondere gelöst in einer alkalischen Flüssigformulierung eingesetzt werden. Es kann insbesondere vorgesehen werden, nach der Reduktion (insbesondere mit Borhydrid) eine Destillation (insbesondere eine fraktionierte Destillation außerdem insbesondere eine Molekulardestillation mit einer möglichst kurzen thermischen Belastung des Glycerins) durchzuführen. Weiterhin kann vor oder nach der Reduktion eine Behandlung des Glycerins bzw. Roh-Glycerins mit Silikaten vorgesehen werden. Durch die Behandlung des Glycerins bzw. Roh-Glycerins mit Silikaten können Metallspuren entfernt werden.

Eine Behandlung mit einem Borhydrid kann insbesondere wie folgt durchgeführt werden. Die Einsatzmenge der Borhydrid-Lösung kann z. B. 100 - 5000 ppm, insbesondere 250 - 2000 ppm, bezogen auf das zu behandelnde Roh-Glycerin betragen. Die Temperatur bei der Behandlung kann 50 bis 90°C betragen. Der pH-Wert bei der Behandlung kann 7 oder größer sein. Die Behandlungsdauer kann 2 Minuten bis 12 Stunden betragen. Die Behandlung kann unter Stickstoffatmosphären erfolgen. Die Entfernung eines evtl. verbleibenden Überschusses an Borhydrid kann durch Ansäuern und/oder durch Destillation erfolgen, insbesondere kann sie durch Destillation erfolgen. Beim Ansäuern erhält man Borat im Produkt, was oft auch nicht gewünscht ist, deshalb wird oft die Entfernung des Bors über Destillation oder alternativ Adsorption an einen Borat-spezifischen Adsorber betrieben.

Folgende Prozessvarianten können für die Durchführung des erfindungsgemäßen Verfahrens vorgesehen werden:
- ein Batch-Prozess nach fraktionierter Destillation mit getakteten Reaktoren
- ein Konti-Prozess nach fraktionierter Destillation in einem Rohrreaktor mit Mischern
- ein Konti-Prozess mit Borhydrid-Festbett in der Gasphase eingebaut zwischen einem erstem Dünnschichtverdampfer und einer Fraktionierkolonne

Das erfindungsgemäße Verfahren kann weiterhin eine Silikat-Behandlung vorsehen. Diese kann wie folgt durchgeführt werden:
- Die Einsatzmenge an Silikat kann 100 bis 10000 ppm, insbesondere 500 - 5000 ppm, bezogen auf das zu behandelnde Glycerin bzw. Roh-Glycerin betragen.
- Die Temperatur bei der Behandlung kann 50 - 90 °C betragen.
- Die Behandlung kann in Kombination mit einer Borhydrid-Behandlung, entweder gleichzeitig oder nacheinander geschaltet, erfolgen.

Wenn das erfindungsgemäße Verfahren eine Destillation umfasst, dann kann diese wie folgt durchgeführt werden:
- als Dünnschichtdestillation oder in einem Fallfilmverdampfer, insbesondere mit kurzer thermischer Belastung
- bei einem Druck von kleiner als 2 mbar
- bei einer Temperatur von weniger als 180 °C
- Nach der Destillation kann eine Entspannung mit Stickstoff erfolgen.
- Die weitere Lagerung und Abfüllung kann unter Stickstoff erfolgen.

Der Gehalt an Aldehyden wird in Glycerin üblicherweise nach Pharmacopeia mit Pararosanilinium Hydrochlorid bestimmt. Es konnte nun im Verlauf der Arbeiten, die der vorliegenden Erfindung zu Grunde liegen, gezeigt werden, dass dieser Test sehr spezifisch für Formaldehyd ist, allerdings andere Aldehyde, die Oxidationsprodukte von Glycerin sind (z. B. Glycerinaldehyd oder Dihydroxyaceton), nicht quantitativ erfasst werden (siehe Beispiele der vorliegenden Schrift).

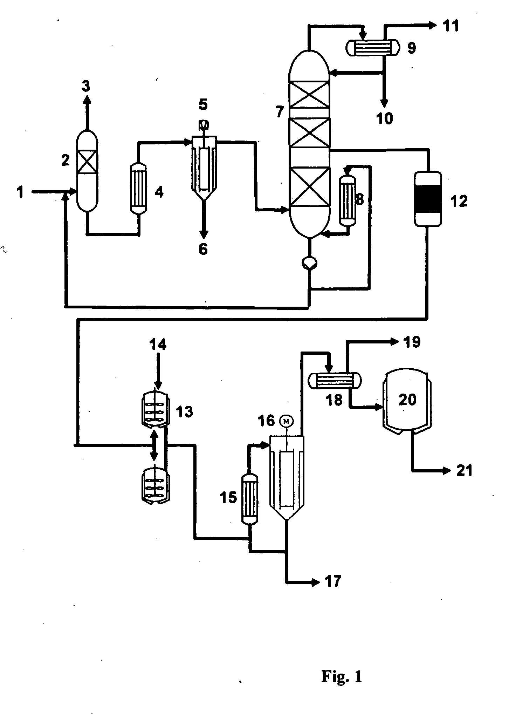

Fig. 1 stellt eine Vorrichtung zur Aufreinigung von Glycerin dar. In dieser Vorrichtung kann Roh-Glycerin mit Borhydrid in einem Batch-Prozess behandelt werden. Die Bezugszeichen haben folgende Bedeutung:
- 1:: Rohglycerin nach Abtrennung der Ölphase
- 2:: Trockner
- 3:: Vakuumanlage, Abtrennung Leichtsieder / Wasser
- 4:: Heizsystem
- 5:: Dünnschichtverdampfer
- 6:: Sumpfprodukt (Salze + Schwersieder)
- 7:: Fraktionierkolonne
- 8:: Reboiler-System
- 9:: Kondensationssystem
- 10:: Kopfprodukt (Leichtsieder)
- 11:: Vakuumsystem
- 12:: Aktivkohle-Behandlung (optional als Säule oder als Batch-Reaktor)
- 13:: 2 Rührkessel zur Borhydrid-Behandlung getaktet
- 14:: Borhydrid-Dosierung
- 15:: Heizsystem
- 16:: Dünnschichtverdampfer (optional Fallfilmverdampfer)
- 17:: Sumpfprodukt (Schwersieder)
- 18:: Kondensationssystem
- 19:: Vakuumsystem
- 20:: Lagertank für Reinglycerin
- 21:: Abfüllung

Mit der beschriebenen Vorrichtung kann ein Glycerin frei von Aldehyden und Ketonen mit einer Lagerstabilität von > 8 Wochen ohne Neubildung von Aldehyd- und Ketonkörpern produziert werden.

Alternativ besteht die Möglichkeit nach der Borhydrid-Behandlung 13 das Produkt erneut über den Dünnschichtverdampfer 5 zu fahren und das Kopfprodukt zu kondensieren. Bei dieser Fahrweise wird der Eingangsstrom in den Dünnschichtverdampfer 5 in Intervallen gewechselt von Rohglycerin zu aufgereinigtem Glycerin. Diese Verfahrensvariante benötigt einen weiteren Puffertank. Die Anlagenkomponenten 15 - 19 können entfallen.

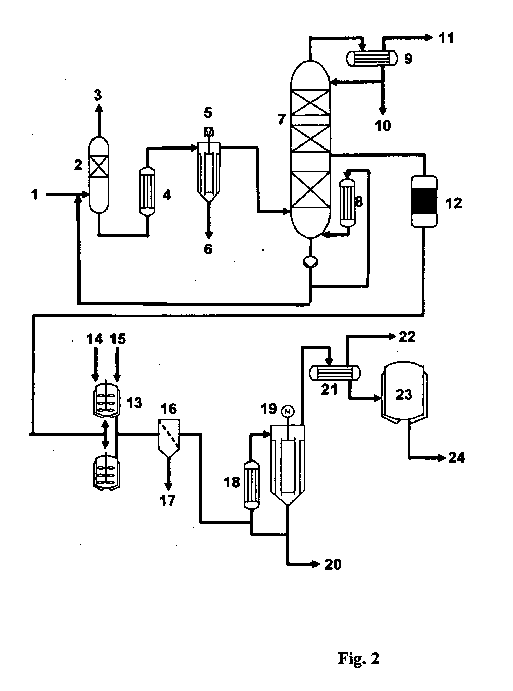

Fig. 2 stellt eine Vorrichtung zur Aufreinigung von Glycerin dar. In dieser Vorrichtung kann Roh-Glycerin kombiniert mit Borhydrid und Silikat in einem Batch-Prozess behandelt werden. Die Bezugszeichen haben folgende Bedeutung:
- 1:: Rohglycerin nach Abtrennung der Ölphase
- 2:: Trockner
- 3:: Vakuumanlage, Abtrennung Leichtsieder / Wasser
- 4:: Heizsystem
- 5:: Dünnschichtverdampfer
- 6:: Sumpfprodukt (Salze + Schwersieder)
- 7:: Fraktionierkolonne
- 8:: Reboiler-System
- 9:: Kondensationssystem
- 10:: Kopfprodukt (Leichtsieder)
- 11:: Vakuumsystem
- 12:: Aktivkohle-Behandlung (optional als Säule oder als Batch-Reaktor)
- 13:: 2 Rührkessel zur Borhydrid-und Silikat-Behandlung getaktet
- 14:: Borhydrid-Dosierung
- 15:: Silikat-Dosierung
- 16:: Silikat Filtration
- 17:: Feststoffabfall
- 18:: Heizsystem
- 19:: Dünnschichtverdampfer (optional Fallfilmverdampfer)
- 20:: Sumpfprodukt (Schwersieder)
- 21:: Kondensationssystem
- 22:: Vakuumsystem
- 23:: Lagertank für Reinglycerin
- 24:: Abfüllung

Mit der beschriebenen Vorrichtung kann ein Glycerin frei von Aldehyden und Ketonen mit einer Lagerstabilität von > 8 Wochen ohne Neubildung von Aldehyd- und Ketonkörpern produziert werden.

Alternativ besteht die Möglichkeit nach der Silikat-Filtration 16 das Produkt erneut über den Dünnschichtverdampfer 5 zu fahren und das Kopfprodukt zu kondensieren. Bei dieser Fahrweise wird der Eingangsstrom in den Dünnschichtverdampfer 5 in Intervallen gewechselt von Rohglycerin zu aufgereinigtem Glycerin. Diese Verfahrensvariante benötigt einen weiteren Puffertank. Die Anlagenkomponenten 18 - 22 können entfallen.

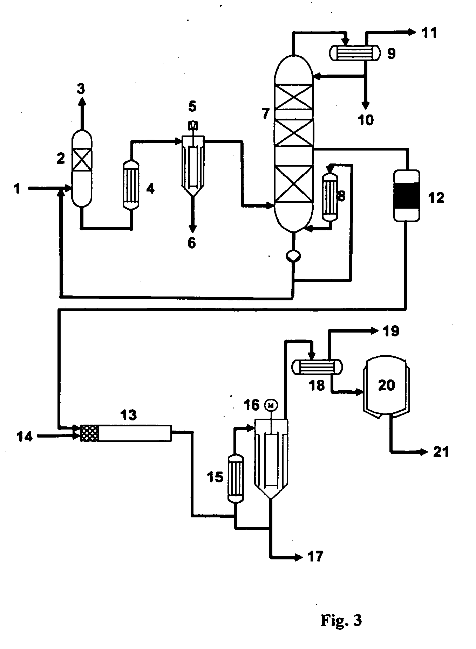

Fig. 3 stellt eine Vorrichtung zur Aufreinigung von Glycerin dar. In dieser Vorrichtung kann Roh-Glycerin kontinuierlich mit Borhydrid in Flüssigphase behandelt werden. Die Bezugszeichen haben folgende Bedeutung:
- 1:: Kohglycerin nach Abtrennung der Ölphase
- 2:: Trockner
- 3:: Vakuumanlage, Abtrennung Leichtsieder / Wasser
- 4:: Heizsystem
- 5:: Dünnschichtverdampfer
- 6:: Sumpfprodukt (Salze + Schwersieder)
- 7:: Fraktionierkolonne
- 8:: Reboiler-System
- 9:: Kondensationssystem
- 10:: Kopfprodukt (Leichtsieder)
- 11:: Vakuumsystem
- 12:: Aktivkohle-Behandlung (optional als Säule oder als Batch-Reaktor)
- 13:: Rohrreaktor zur Borhydrid-Reduzierung mit eingebautem Mikromischer oder alternativ mit eingebauten statischen Mischern
- 14:: Borhydrid-Dosierung
- 15:: Heizsystem
- 16:: Dünnschichtverdampfer (optional Fallfilmverdampfer)
- 17:: Sumpfprodukt (Schwersieder)
- 18:: Kondensationssystem
- 19:: Vakuumsystem
- 20:: Lagertank für Reinglycerin
- 21:: Abfüllung

Mit der beschriebenen Vorrichtung kann ein Glycerin frei von Aldehyden und Ketonen mit einer Lagerstabilität von > 8 Wochen ohne Neubildung von Aldehyd- und Ketonkörpern produziert werden.

Alternativ besteht die Möglichkeit nach der Borhydrid-Behandlung 13 das Produkt erneut über den Dünnschichtverdampfer 5 zu fahren und das Kopfprodukt zu kondensieren. Bei dieser Fahrweise wird der Eingangsstrom in den Dünnschichtverdampfer 5 in Intervallen gewechselt von Rohglycerin zu aufgereinigtem Glycerin. Diese Verfahrensvariante benötigt einen weiteren Puffertank. Die Anlagenkomponenten 15 - 19 können entfallen.

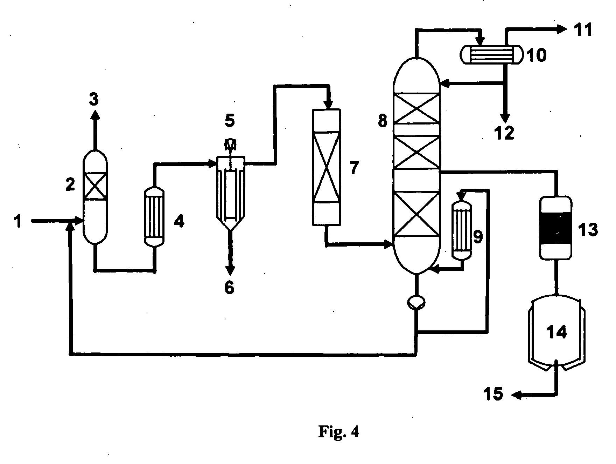

Fig. 4 stellt eine Vorrichtung zur Aufreinigung von Glycerin dar. In dieser Vorrichtung kann Roh-Glycerin mit Borhydrid in der Gasphase behandelt werden gekoppelt mit einer Destillation. Die Bezugszeichen haben folgende Bedeutung:
- 1:: Rohglycerin nach Abtrennung der Ölphase
- 2:: Trockner
- 3:: Vakuumanlage, Abtrennung Leichtsieder / Wasser
- 4:: Heizsystem
- 5:: Dünnschichtverdampfer
- 6:: Sumpfprodukt (Salze + Schwersieder)
- 7:: Reaktor zur Borhydridbehandlung mit Borhydrid-Festbett: Glycerin wird gasförmig durch das Festbett geleitet.
- 8:: Fraktionierkolonne
- 9:: Reboiler-System
- 10:: Kondensationssystem
- 11:: Vakuumsystem
- 12:: Kopfprodukt (Leichtsieder)
- 13:: Aktivkohle-Behandlung (optional als Säule oder als Batch-Reaktor)
- 14:: Lagertank für Reinglycerin
- 15:: Abfüllung

Mit der beschriebenen Vorrichtung kann ein Glycerin frei von Aldehyden und Ketonen mit einer Lagerstabilität von > 8 Wochen ohne Neubildung von Aldehyd- und Ketonkörpern produziert werden.

Im Unterschied zu den in den Vorrichtungen gemäß Fig. 1 bis 3 wird in dieser Anlage die Borhydridbehandlung in teilgereinigtem Glycerin durchgeführt. Die notwendige Borhydridkonzentration ist bei diesem Verfahren höher.

Zweckmäßig wird die Anlage mit 2 Borhydrid-Festbetten kontinuierlich betrieben. Dabei ist immer ein Festbett in Betrieb, während im anderen Festbett die Borhydrid-Füllung erneuert werden kann.

### Beispiele

### Beispiel 1: Vergleichende Glycerinanalytik

### A) Pararosalinium Farbtest

Formaldehyd, Propionaldehyd, Glycerinaldehyd, Hydroxyaceton und Dihydroxyaceton wurden im Pararosalinium Hydrochlorid Farbtest nach Vorschrift der Pharmacopeia auf ihre Nachweisbarkeit hin bei verschiedenen Konzentrationen überprüft:

| **Substanz** | **Form-** | **Propion-** | **Glycerin-** | **Hydroxy-** | **Dihydroxy-** |
|---|---|---|---|---|---|
| | **aldehyd** | **aldehyd** | **aldehyd** | **aceton** | **aceton** |
| ppm | Abs 552 nm | Abs 552 nm | Abs 562 nm | Abs 562 nm | Abs 552 nm |
| | | | | | |
| 0 | 0 | 0 | 0 | 0 | 0 |
| 3 | 0,06 | nd | nd | nd | nd |
| 6 | 0,14 | nd | nd | nd | nd |
| 9 | 0,29 | nd | nd | nd | nd |
| 15 | 0,74 | nd | nd | nd | nd |
| 30 | 3,8 | 0,07 | 0,02 | 0,02 | 0,02 |
| 100 | > limit | 0,99 | 0,04 | 0,02 | 0,02 |
| 300 | > limit | > limit | 0,18 | 0,02 | 0,02 |

| | | | | | |
|---|---|---|---|---|---|
| Es bedeuten: Abs = Absorbtion (bei einer Wellenlänge von 552 nm im Photometer); limit = Das Absorbtionslimit des Photometers, das bei 4 liegt n.d. = not determined, d. h. nicht bestimmt | | | | | |

Fazit: Der Farbtest besitzt nur eine hohe Empfindlichkeit für Formaldehyd. Der Test ist nicht geeignet zum Nachweis anderer, häufig im Glycerin enthaltener Aldehyde und Ketone wie zum Beispiel der direkten Oxidationsprodukte Glycerinaldehyd und Dihydroxyaceton sowie das durch Umlagerung herstellbare Hydroxyaceton.

### B) HPLC Analytik

Verschiedene Aldehyde und Ketone wurden mit 2,4-Dinitrophenylhydrazin derivatisiert und die entstehenden Hydrazonverbindungen wurden mit Hilfe einer HPLC in einem Phosphorsäure / Acetonitril Gradienten getrennt und mit einem Dioden Array bei 340 nm analysiert. Zur Trennung wurde eine Reversed Phase Säule (Reprosil Pur C18 AQ) eingesetzt. Folgende Nachweisgrenzen wurden ermittelt:

| **Substanz** | **Nachweis-** |
|---|---|
| | **grenze** |
| Formaldehyd | 0,5 |
| Acetaldehyd | 0,5 |
| Acrolein | 0,5 |
| Aceton | 0,5 |
| Propanal | 0,5 |
| Butanal | 0,5 |
| Glycerinaldehyd | 2 |
| Dihydroxyaceton | 2 |
| Hydroxyaceton | 2 |
| Malondialdehyd | 10 |
| Glyoxal/Benzald | 10 |
| Hexanal | 10 |
| Oktanal | 10 |
| Dekanal | 10 |
| Dodekanal | 10 |
| Tetradekanal | 10 |
| Hexadekanal | 10 |

Fazit: Die HPLC Methode ist sehr gut geeignet verschiedenste Aldehyde und Ketone in Glycerin in Spuren zu analysieren. Für alle weiteren Tests wurde die HPLC Analytik verwendet.

### Beispiel 2: Vergleich der Rohglycerin-Qualität hergestellt über verschiedene oleochemische Prozesse

Verschiedene Rohglycerine wurden auf ihren Gehalt an Aldehyden und Ketonen hin untersucht. Dabei wurden Glycerine aus enzymatischer Hydrolyse (Probe A aus Technikumsmaßstab); aus Hochdruck-Ölspaltung (Probe B aus Produktionsmaßstab), aus alkalischer Niederdruckumesterung (Probe C aus Produktionsmaßstab), aus Zink-katalysierter Hochdruckumesterung (Probe D aus Produktionsmaßstab und aus katalysatorfreier Hochdruck- und Hochtemperaturumesterung (Probe E aus Technikumsmaßstab) analysiert. < bedeutet unterhalb der entsprechenden Nachweisgrenze wie in Beispiel 1 spezifiziert.

| | **A** | **B** | **C** | **D** | **E** |
|---|---|---|---|---|---|
| **Substanz** | **ppm** | **ppm** | **ppm** | **ppm** | **ppm** |
| | | | | | |
| Formaldehyd | 0,6 | 4,9 | 6,7 | 12,2 | 130 |
| Acetaldehyd | < | 1,1 | 0,7 | 2,4 | 13 |
| Acrolein | 2,5 | 1,8 | < | 1,3 | 9 |
| Aceton | < | 0,6 | < | < | 14 |
| Propanal | 2,9 | 6,2 | 2,2 | 0,7 | 9,6 |
| Butanal | < | 5,8 | < | 0,9 | 1,2 |
| Glycerinaldehyd (GA) | 2,8 | 12 | 9 | 5 | 60 |
| Dihydroxyaceton (DHA) | 2,5 | 18 | 6 | 8 | 120 |
| Hydroxyaceton (HA) | 2,3 | 51 | < | 18 | 420 |
| Malondialdehyd | < | 40 | < | < | 120 |
| Glyoxal/Benzald | < | 28 | < | 11 | 260 |
| Hexanal | < | < | < | < | < |
| Oktanal | < | < | < | < | < |
| Dekanal | < | < | < | < | < |
| Dodekanal | < | < | < | < | < |
| Tetradekanal | < | < | < | < | < |
| Hexadekanal | < | < | < | < | < |
| | | | | | |
| **Summe Aldehyde / Ketone** | **13,6** | **169,4** | **24,6** | **59,5** | **1156,8** |
| **Summe GA, DHA, HA** | **7,6** | **81** | **15** | **31** | **600** |

Fazit: Die Glycerine aus den Prozessen, die mit niedriger Temperatur druckfrei betrieben werden, haben die beste Ausgangsqualität.

### Beispiel 3: Vergleich der Rohglycerin-Qualität verschiedener pflanzlicher Rohstoffquellen

Rohglycerine basierend auf unterschiedlichen Pflanzenölen wurden im Produktionsmaßstab über alkalische Niederdruckumesterung umgesetzt und die Glycerine wurden direkt nach der Spaltung (A-Proben) sowie nach der Methanolentfernung und Entfettung (B-Proben) analysiert. Folgende Rohstoffquellen wurden untersucht: High Erucic Raps (1A + 1B), Low Erucic Raps (2A + 2B) sowie Palmöl (3A + 3B). < bedeutet unterhalb der entsprechenden Nachweisgrenze wie in Beispiel 1 spezifiziert.

| | **1A** | **1B** | **2A** | **2B** | **3A** | **3B** |
|---|---|---|---|---|---|---|
| **Substanz** | **ppm** | **ppm** | **ppm** | **ppm** | **ppm** | **ppm** |
| | | | | | | |
| Formaldehyd | 2,8 | 0,5 | 4,5 | 0,7 | 4,9 | 0,9 |
| Acetaldehyd | 6,4 | 0,7 | 4,7 | 0,7 | 9,2 | 1,1 |
| Acrolein | < | < | < | < | < | < |
| Aceton | 6 | < | 4,1 | < | 12,8 | < |
| Propanal | 10,4 | 1,8 | 7,2 | 0,7 | 12,4 | 3 |
| Butanal | 2,4 | < | 1,4 | < | 3,2 | < |
| Glycerinaldehyd (GA) | 3 | 4 | 4 | 6 | 24 | 6 |
| Dihydroxyaceton (DHA) | < | 3 | 2 | 4 | 10 | 7 |
| Hydroxyaceton (HA) | 2 | 11 | < | < | 4 | < |
| Malondialdehyd | 21 | < | < | < | 15 | < |
| Glyoxal/Benzald | < | < | < | < | 16 | < |
| Hexanal | < | < | < | < | 10 | < |
| Oktanal | < | < | < | < | < | < |
| Dekanal | < | < | < | < | < | < |
| Dodekanal | < | < | < | < | < | < |
| Tetradekanal | < | < | < | < | < | < |
| Hexadekanal | < | < | < | < | < | < |
| | | | | | | |
| **Summe Aldehyde / Ketone** | **54** | **21** | **27,9** | **12,1** | **121,5** | **18** |
| **Summe GA, DHA, HA** | **5** | **18** | **6** | **10** | **38** | **13** |

Fazit: Low erucic Raps ist gut als Rohstoffquelle geeignet. Palmöl als Rohstoffquelle führt zur höchsten Konzentration an verunreinigenden Aldehyden und Ketonen.

### Beispiel 4: Vergleich der Reinglycerinqualität aus verschiedenen Aufarbeitungsprozessen

Verglichen wurden die Aldehyd- und Ketongehalt von Reinglycerin chemisch hergestellt aus Propylen (Probe A), pflanzlich basiertes Glycerin aufgereinigt über fraktionierte Destillation (Probe B + C, zwei separate Chargen aus Produktionsmaßstab) sowie pflanzlich basiertes Glycerin nicht destillativ aufgereinigt über Ionentauscher und absorptive Harze (Probe D aus Produktionsmaßstab). Alle untersuchten Glycerine erfüllten die Pharmacopeia Richtlinien.

| | **A** | **B** | **C** | **D** |
|---|---|---|---|---|
| **Substanz** | **ppm** | **ppm** | **ppm** | **ppm** |
| | | | | |
| Formaldehyd | < | < | < | 5 |
| Acetaldehyd | < | < | < | < |
| Acrolein | < | < | < | < |
| Aceton | < | < | < | < |
| Propanal | < | < | < | < |
| Butanal | < | < | < | < |
| Glycerinaldehyd (GA) | < | < | 2,5 | 8,3 |
| Dihydroxyaceton (DHA) | < | < | < | 10 |
| Hydroxyaceton (HA) | < | < | < | 1,9 |
| Malondialdehyd | < | < | < | 15 |
| Glyoxal/Benzald | < | < | < | < |
| Hexanal | < | < | < | < |
| Oktanal | < | < | < | < |
| Dekanal | < | < | < | < |
| Dodekanal | < | < | < | < |
| Tetradekanal | < | < | < | < |
| Hexadekanal | < | < | < | < |
| | | | | |
| **Summe Aldehyde / Ketone** | **0** | **0** | **2,5** | **40,2** |
| **Summe GA, DHA, HA** | **0** | **0** | **2,5** | **20,2** |

Fazit: Über fraktionierte Destillation aufgereinigtes Glycerin hat eine gute mit chemisch aus Propylen vergleichbare Qualität. Über Chromatographie gereinigtes Glycerin enthält dagegen noch Aldehyd- und Ketonverunreinigungen, die über den Pararosalinium Hydrochlorid Farbtest nicht quantitativ detektiert werden.

### Beispiel 5: Hydrierung und Destillation von Rohglycerin

600 g der jeweiligen Rohglycerine wurden mit 30 g Wasser und 0,6 g einer alkalischen Borhydrid-Lösung mit einem Borhydridgehalt von 20 Gew.% versetzt und für 2 h bei 80 °C unter Stickstoffatmosphäre inkubiert. Anschließend wurde das Wasser unter Vakuum abdestilliert und die getrockneten Glycerine wurden einer Kurzwegdestillation bei einer Temperatur von 150 °C unterzogen. Die Destillationsapparatur wurde mit Stickstoff belüftet und die Glycerine wurden unter Stickstoff abgefüllt. Für die Versuche wurden Rohglycerine aus der enzymatischen Hydrolyse (Beispiel 2, Probe A) und aus der alkalischen Niederdruckumesterung (Beispiel 2, Probe C) eingesetzt. Die Proben wurden einem Lagertest unterzogen (siehe folgende Beispiele).

Ergebnis: Die in den Proben vorhandenen Aldehyde und Ketone konnten deutlich reduziert werden. Die Hydrierung funktionierte besser bei dem Glycerin aus der enzymatischen Hydrolyse. Grund hierfür war eine niedrigere Konzentration an Verunreinigungen, die das Borhydrid verbrauchen.

### Beispiel 6: Hydrierung und Destillation von Reinglycerin

600 g der jeweiligen Reinglycerine wurden mit 30 g Wasser und 0,6 g einer alkalischen Borhydrid-Lösung mit einem Borhydridgehalt von 20 Gew.% versetzt und für 2 h bei 80 °C unter Stickstoffatmosphäre inkubiert. Anschließend wurde das Wasser unter Vakuum abdestilliert und die getrockneten Glycerine wurden einer Kurzwegdestillation bei einer Temperatur von 150 °C destilliert. Die Destillationsapparatur wurde mit Stickstoff belüftet und die Glycerine wurden unter Stickstoff abgefüllt. Für die Versuche wurden die Reinglycerine aus Beispiel 4, Proben B und C, eingesetzt. Die Proben wurden einem Lagertest unterzogen (siehe folgende Beispiele).

Ergebnis: In beiden Mustern waren keine Aldehyde oder Ketone über HPLC detektierbar.

### Beispiel 7: Hydrierung und Destillation von Reinglycerin

600 g Reinglycerin (Probe B aus Beispiel 4) wurden mit 30 g Wasser und 0,6 g einer alkalischen Borhydrid-Lösung mit einem Borhydridgehalt von 20 Gew.% versetzt und für 2 h bei 80 °C unter Stickstoffatmosphäre inkubiert. Nach der Reaktion wurde überschüssiges Borhydrid durch Ansäuern mit Salzsäure auf pH 5 zerstört. Anschließend wurde das Wasser unter Vakuum abdestilliert und die getrockneten Glycerine wurden einer Kurzwegdestillation bei einer Temperatur von 150 °C destilliert. Die Destillationsapparatur wurde mit Stickstoff belüftet und die Glycerine wurden unter Stickstoff abgefüllt. Die Probe wurde einem Lagertest unterzogen (siehe folgende Beispiele).

Ergebnis: Im hergestellten Muster waren keine Aldehyde oder Ketone über HPLC detektierbar.

### Beispiel 8: Vergleich der Glycerinqualität der hydrierten und destillierten Glycerine

Verglichen wurden die in den Beispielen 5, 6 und 7 hergestellten Muster mit den entsprechenden Ausgangssubstanzen: A: Glycerin aus Beispiel 2, Probe A; B: Muster A hydriert (Beispiel 5); C: Glycerin aus Beispiel 2, Probe C; D: Muster C hydriert (Beispiel 5); E: Glycerin aus Beispiel 4, Probe B; F: Muster E hydriert (Beispiel 6); G: Muster E hydriert und neutralisiert (Beispiel 7); H: Glycerin aus Beispiel 4, Probe C; I: Muster H hydriert (Beispiel 6).

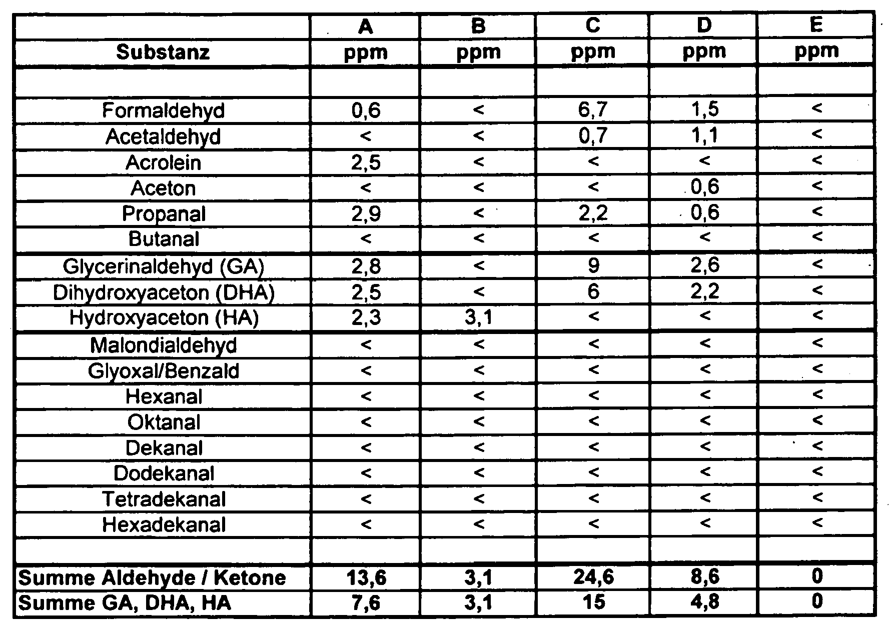

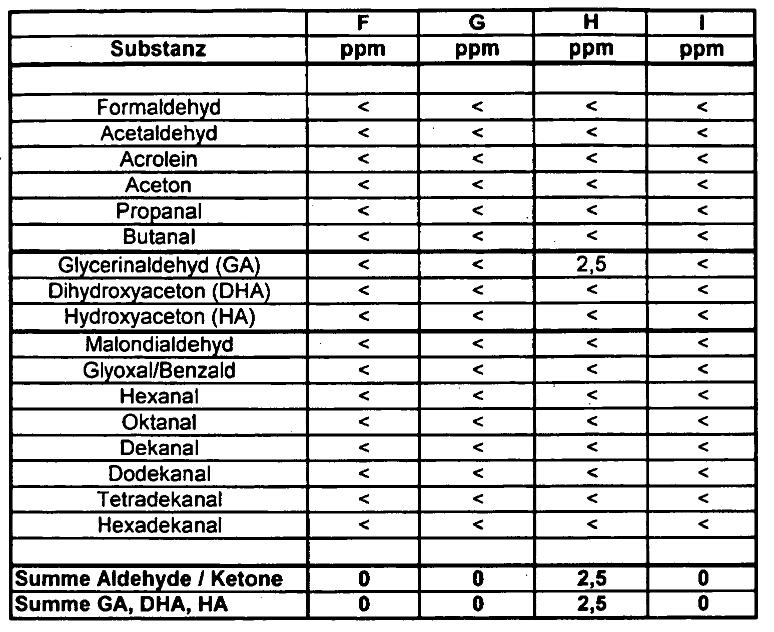

Fazit: Die Hydrierung reduziert die vorhandenen Aldehyde und Ketone deutlich. Durch die nachfolgende Destillation entstehen keine neuen oxidierten Nebenkomponenten.

### Beispiel 9: Konzentrationsabhängigkeit der Reduktion in Glycerin mit Borhydrid

Reinglycerin wurde mit 10 % Wasser und gleichen Teilen Formaldehyd, Propionaldehyd, Glycerinaldehyd, Dihydroxyaceton und Hydroxyaceton versetzt. Die Summe der detektierbaren Aldehyde betrug 152 ppm. Es wurde Borhydrid-Lösung in Konzentrationen von 500 ppm, 1500 ppm und 5000 ppm zugegeben und 2 h bei 80 °C inkubiert. Anschließend wurden die Gemische mit Zitronensäure auf pH 5 eingestellt und analysiert.

Ergebnis: Bereits bei einer Konzentration von 500 ppm wurden die Aldehyde und Ketone quantitativ reduziert und waren nicht mehr detektierbar.

### Beispiel 10: Konzentrationsabhängigkeit der Reduktion in Glycerin mit Borhydrid

Ein Reinglycerin geringerer Qualität wurde mit 10 % Wasser und gleichen Teilen Formaldehyd, Propionaldehyd, Glycerinaldehyd, Dihydroxyaceton und Hydroxyaceton versetzt. Die Summe der detektierbaren Aldehyde betrug 44 ppm. Es wurde Borhydrid-Lösung in Konzentrationen von 400 ppm, 1000 ppm, 2000 ppm und 5000 ppm zugegeben und 2 h bei 80 °C inkubiert. Anschließend wurden die Gemische mit Salzsäure auf pH 5 eingestellt und analysiert.

Ergebnis: Bis zu einer Konzentration von 1000 ppm waren Aldehyde und Ketone detektierbar, ab 2000 ppm Borhydrid-Lösung wurden die Aldehyde und Ketone quantitativ reduziert und waren nicht mehr detektierbar.

### Beispiel 11: Konzentrationsabhängigkeit der Reduktion in Glycerin mit Borhydrid

Methanolhaltiges Rohglycerin aus einer alkalischen Niederdruckumesterung mit einem Gesamtgehalt an detektierbaren Aldehyden und Ketonen von 188 ppm wurde mit 500 ppm und 2000 ppm Borhydrid-Lösung versetzt und 2 h bei 80 °C inkubiert. Anschließend wurde das Methanol abdestilliert und das Rohglycerin analysiert.

Ergebnis: Auch bei 2000 ppm Borhydrid-Lösung konnte im Vergleich zur unbehandelten Probe keine signifikante Abnahme der Aldehyd- und Ketonkonzentration beobachtet werden.

### Beispiel 12: Einfluss des Wassergehaltes auf die Reduktion mit Borhydrid in Glycerin

Die Borhydridbehandlung des Reinglycerins wurde analog zu Beispiel 9 wiederholt mit dem Unterschied, dass kein Wasser zum Glycerin gegeben wurde und dass die Aldehyde und Ketone direkt im Glycerin gelöst wurden. Im Reinglycerin wurde eine Gesamtkonzentration von 114 ppm Aldehyden und Ketonen detektiert.

Ergebnis: Auch im wasserfreien Glycerin waren bereits ab einer Konzentration von 500 ppm Borhydrid-Lösung alle Aldehyde und Ketone quantitativ reduziert.

### Beispiel 13: Vergleichende Analyse der Beispiele 9 - 12

Die Reinheit des Glycerins hatte einen starken Einfluss auf die Wirkung des Borhydrids. Je mehr Verunreinigungen im Glycerin vorhanden waren, desto schlechter ist die Wirkung des Borhydrids, was auf chemische Verbindungen zurückzuführen ist, die das Borhydrid verbrauchten.

Qualitativ hochwertige Ausgangsglycerine sind daher am besten für die nachgeschaltete reduktive Aufreinigung geeignet.

Der Wassergehalt im Glycerin hat keinen signifikanten Einfluss auf die Borhydrid katalysierte Reduktion.

### Beispiel 14: Aldehyd- und Ketonbildung bei der Glycerinbehandlung mit Silikaten

Ein Reinglycerin wurde mit 0,1 % Silikat (Trisyl) versetzt und 1 h bei 60 °C inkubiert. Das gleiche Reinglycerin wurde mit 0,1 % eines weiteren Silikates (Magnesol) versetzt und bei 140 °C für 1 h inkubiert. Nach der Silikatbehandlung wurden die Silikate vom Glycerin über Filtration entfernt und die Glycerine wurden gegen die Ausgangssubstanz als Referenz auf ihren Aldehyd- und Ketongehalt hin verglichen.

| | **A** | **B** | **C** |
|---|---|---|---|
| **Substanz** | **ppm** | **ppm** | **ppm** |
| | | | |
| Formaldehyd | < | 0,5 | 6,6 |
| Acetaldehyd | < | < | 0,5 |
| Acrolein | < | < | 4,9 |
| Aceton | < | < | 4,3 |
| Propanal | < | < | 2,7 |
| Butanal | < | < | 0,5 |
| Glycerinaldehyd (GA) | 4,4 | 5,1 | 6,2 |
| Dihydroxyaceton (DHA) | 3,6 | 3,4 | 9,2 |
| Hydroxyaceton (HA) | < | < | 61 |
| Malondialdehyd | < | < | < |
| Glyoxal/Benzald | < | < | 10 |
| Hexanal | < | < | < |
| Oktanal | < | < | < |
| Dekanal | < | < | < |
| Dodekanal | < | < | < |
| Tetradekanal | < | < | < |
| Hexadekanal | < | < | < |
| | | | |
| **Summe Aldehyde / Ketone** | **8** | **9** | **105,9** |
| **Summe GA, DHA, HA** | **8** | **8,5** | **76,4** |

Fazit: Die Behandlung mit aktivierten Silikaten bei niedriger Temperatur ist zur Entfernung von Metallspuren geeignet. Dagegen wirkt sich eine Silikatbehandlung bei hohen Temperaturen sehr nachteilig auf die Aldehyd- und Ketonkonzentration aus.

### Beispiel 15: Oxidationsstabilität verschiedener Rohglycerine während der Lagerung im Vergleich zu Glycerin hergestellt aus Propylen

Rohglycerine aus Beispiel 2, Proben A und C wurden einem Lagertest unterzogen. Dabei wurden die Muster jeweils in Glas- und in Plastikflaschen abgefüllt (bei den Glasflaschen handelte es sich um übliche transparente Glasflaschen, die für die Lagerung von Chemikalien im Labor verwendet werden) und bei einer konstanten Temperatur von 40 °C gelagert (im Dunkeln in einem Wärmeschrank). Alle 4 Wochen wurde eine Probe entnommen und analysiert. In der folgenden Tabelle ist die Summe aus Glycerinaldehyd und Dihydroxyaceton dargestellt, die die direkten Oxidationsprodukte ausgehend von Glycerin sind:
A: Reinglycerin aus chemischer Herstellung (aus Beispiel 4, Probe A);
B: Rohglycerin aus enzymatischer Spaltung (aus Beispiel 2, Probe A);
C: Rohglycerin aus alkalischer Umesterung (Beispiel 2, Probe C).

| | **Start** | **4 Wochen** | **4 Wochen** | **8 Wochen** | **8 Wochen** |
|---|---|---|---|---|---|
| | **Lagerung** | **Plastik** | **Glas** | **Plastik** | **Glas** |
| **Probe** | **ppm GA+ DHA** | **ppm GA + DHA** | **ppm GA + DHA** | **ppm GA + DHA** | **ppm GA+ DHA** |
| | | | | | |
| A | 0 | 2,6 | 2,6 | 9,6 | 9,2 |
| B | 9,1 | 34 | 23 | 54 | 43 |
| C | 27 | 34 | 76 | 51 | 26 |

Fazit: Die Rohglycerine weisen eine stärkere Aldehyd- und Ketonbildung in der Lagerung auf als das Reinglycerin.

### Beispiel 16: Oxidationsstabilität von Reinglycerinen während der Lagerung im Vergleich zu Glycerin hergestellt aus Propylen

Reinglycerine aus Beispiel 4 Proben A bis C wurden einem Lagertest unterzogen. Dabei wurden die Muster jeweils in Glas- und in Plastikflaschen abgefüllt und bei einer konstanten Temperatur von 40 °C gelagert. Alle 4 Wochen wurde eine Probe entnommen und analysiert. In der folgenden Tabelle ist die Summe aus Glycerinaldehyd und Dihydroxyaceton dargestellt, die die direkten Oxidationsprodukte ausgehend von Glycerin sind.

| | **Start** | **4 Wochen** | **4 Wochen** | **8 Wochen** | **8 Wochen** |
|---|---|---|---|---|---|
| | **Lagerung** | **Plastik** | **Glas** | **Plastik** | **Glas** |
| **Probe** | **ppm GA + DHA** | **ppm GA + DHA** | **ppm GA+DHA** | **ppm GA + DHA** | **ppm GA + DHA** |
| | | | | | |
| A | 0 | 2,6 | 2,6 | 9,6 | 9,2 |
| B | 0 | 6,3 | 6,1 | 10,3 | 9,9 |
| C | 2,5 | 7,7 | 8,3 | 12,8 | 11.9 |

Fazit: Auch die Reinglycerine waren nicht lagerstabil. Über 8 Wochen Lagerung war ein deutlicher Anstieg der Aldehyde und Ketone zu beobachten.

### Beispiel 17: Oxidationsstabilität von hydriertem Rohglycerin während der Lagerung im Vergleich zu unbehandeltem Rohglycerin

Die hydrierten Rohglycerine aus Beispiel 5 wurden gegen die entsprechenden Ausgangsglycerine im Lagertest verglichen:
A: Rohglycerin aus enzymatischer Hydrolyse;
B: Probe A hydriert und destilliert;
C: Rohglycerin aus alkalischer Niederdruckumesterung;
D: Probe C hydriert und destilliert.

| | **Start** | **4 Wochen** | **4 Wochen** | **8 Wochen** | **8 Wochen** |
|---|---|---|---|---|---|
| | **Lagerung** | **Plastik** | **Glas** | **Plastik** | **Glas** |
| **Probe** | **ppm GA+ DHA** | **ppm GA+ DHA** | **ppm GA+ DHA** | **ppm GA+ DHA** | **ppm GA+ DHA** |
| | | | | | |
| A | 27 | 34 | 76 | 51 | 26 |
| B | 4,8 | 7,2 | 7,8 | 9,2 | 9,4 |
| C | 9,1 | 34 | 23 | 54 | 43 |
| D | 0 | 0 | 0 | 7,8 | 2,2 |

Fazit: Die Lagerstabilität der hydrierten Rohglycerine ist deutlich verbessert.

### Beispiel 18: Oxidationsstabilität von hydriertem Reinglycerin während der Lagerung im Vergleich zu unbehandeltem Reinglycerin

Die hydrierten Reinglycerine aus Beispiel 5 wurden gegen die entsprechenden Ausgangsglycerine im Lagertest verglichen:
A: Reinglycerin aus chemischer Herstellung (Beispiel 4, Probe A);
B: Reinglycerin auf pflanzlicher Basis (Beispiel 4, Probe B);
C: Probe B hydriert und destilliert;
D: Probe B hydriert, angesäuert und destilliert;
E: Reinglycerin auf pflanzlicher Basis (Beispiel 4, Probe C);
F: Probe E hydriert und destilliert.

Fazit: Die Lagerstabilität der hydrierten Reinglycerine war deutlich verbessert. Nach 8 Wochen war keine oder nur eine sehr geringe Bildung an Aldehyden und Ketonen zu verzeichnen. Die hydrierten Glycerine auf pflanzlicher Basis hatten eine bessere Qualität als Reinglycerin hergestellt aus Propylen.

| | **Start** | **4 Wochen** | **4 Wochen** | **8 Wochen** | **8 Wochen** |
|---|---|---|---|---|---|
| | **Lagerung** | **Plastik** | **Glas** | **Plastik** | **Glas** |
| **Probe** | **ppm GA + DHA** | **ppm GA + DHA** | **ppm GA + DHA** | **ppm GA + DHA** | **ppm GA + DHA** |
| | | | | | |
| A | 0 | 2,6 | 2,6 | 9,6 | 9,2 |
| B | 0 | 6,3 | 6,1 | 10,3 | 9,9 |
| C | 0 | 0 | 0 | 0 | 0 |
| D | 0 | 0 | 0 | 0 | 0 |
| E | 2,5 | 7,7 | 8,3 | 12,8 | 11,9 |
| F | 0 | 0 | 0 | 0 | 2,6 |

### Beispiel 19: Oxidationsstabilität von Silikat behandeltem Reinglycerin während der Lagerung im Vergleich zu unbehandeltem Reinglycerin

Die Proben aus Beispiel 14 wurden einem Lagertest unterzogen.

| | **Start** | **4 Wochen** | **4 Wochen** | **8 Wochen** | **8 Wochen** |
|---|---|---|---|---|---|
| | **Lagerung** | **Plastik** | **Glas** | **Plastik** | **Glas** |
| **Probe** | **ppm GA + DHA** | **ppm GA + DHA** | **ppm GA + DHA** | **ppm GA + DHA** | **ppm GA + DHA** |
| | | | | | |
| A | 7,7 | 12 | 13,3 | 18,7 | 18,7 |
| B | 8,5 | 13,7 | 13,7 | 23 | 23 |
| C | 15,4 | 77 | 56 | 115 | 118 |

Fazit: Die Behandlung mit Silikaten bei niedriger Temperatur hatte keinen signifikant negativen Einfluss auf die Neubildung von Aldehyden und Ketonen. Insofern scheint die Behandlung für eine Entfernung von Metallspuren in Kombination mit einer Borhydrid Reduktion geeignet zu sein.

### Beispiel 20: Oxidationsstabilität von Seife gespiktem Reinglycerin während der Lagerung im Vergleich zu unbehandeltem Reinglycerin

200 ppm Kaliumlinolenat wurden in Reinglycerin aus Beispiel 4, Probe B untergemischt. Das mit polyungesättigter Seife gespikte Glycerin (Probe B) wurde im Lagertest gegen die Ausgangssubstanz (Probe A) verglichen.

| | **Start** | **4 Wochen** | **4 Wochen** | **8 Wochen** | **8 Wochen** |
|---|---|---|---|---|---|
| | **Lagerung** | **Plastik** | **Glas** | **Plastik** | **Glas** |
| **Probe** | **ppm GA + DHA** | **ppm GA + DHA** | **ppm GA + DHA** | **ppm GA + DHA** | **ppm GA + DHA** |
| | | | | | |
| A | 0 | 6,3 | 6,1 | 10,3 | 9,9 |
| B | 2,1 | 7,7 | 9,3 | 16,7 | 19,8 |

Fazit: Polyungesättigte Seife katalysierte eine verstärkte Neubildung von Aldehyden und Ketonen.

## Patentansprüche

1. Ein Verfahren zur Herstellung von Glycerin umfassend
• die Bereitstellung eines pflanzlichen Öls oder Fetts,
• die Gewinnung von Roh-Glycerin aus dem pflanzlichen Öl oder Fett und
• die Behandlung des Roh-Glycerins mit einem Reduktionsmittel,
wobei das Reduktionsmittel ein Borhydrid ist und wobei das Verfahren weiterhin umfasst
• die Entfernung des Bors in Form einer Borverbindung aus dem Glycerin durch Destillation nach der Behandlung mit dem Borhydrid.

2. Das Verfahren nach Anspruch 1 weiterhin umfassend
• die Destillation des Roh-Glycerins, bevor es mit dem Reduktionsmittel behandelt wird.

3. Das Verfahren nach Anspruch 2 weiterhin umfassend
• eine Seifenabtrennung aus dem Roh-Glycerin, bevor es mit dem Reduktionsmittel behandelt wird, und
• die Behandlung des Roh-Glycerins mit einem Adsorptionsmittel bevor es mit dem Reduktionsmittel behandelt wird.

4. Das Verfahren nach einem der Ansprüche 1 bis 3, wobei die Behandlung des Roh-Glycerins mit einem Reduktionsmittel bei einem pH-Wert von größer als 7 durchgeführt wird, und wobei die Einstellung dieses pH-Wertes durch die Zugabe einer Base erfolgt.

5. Das Verfahren nach einem der Ansprüche 1 bis 4, wobei das Reduktionsmittel Natriumborhydrid ist, und wobei das Natriumborhydrid in einer Konzentration von 0,005 bis 0,05 Gew.-%, bezogen auf das Roh-Glycerin, eingesetzt wird, und wobei die Behandlung des Roh-Glycerins mit dem Natriumborhydrid bei einer Temperatur von 60 bis 100 °C erfolgt.

6. Das Verfahren nach einem der Ansprüche 1 bis 5, wobei die Gewinnung des Roh-Glycerins aus dem pflanzlichen Öl oder Fett ausschließlich bei Temperaturen von weniger als 100 °C und ausschließlich bei Drücken von weniger als 2 bar (so genanntes Niedertemperatur-Niederdrückverfahren) erfolgt.

7. Ein Verfahren zur Herstellung eines Arzneimittels, wobei das Arzneimittel Glycerin und mindestens einen pharmazeutischen Wirkstoff enthält, und wobei das Verfahren umfasst
a) das Verfahren nach einem der Ansprüche 1 bis 6 und
b) das in Kontakt Bringen des Glycerins mit mindestens einem pharmazeutischen Wirkstoff.

8. Das Verfahren zur Herstellung eines Arzneimittels nach Anspruch 7, wobei das Arzneimittel ein Protein enthält, oder wobei das Arzneimittel eine Verbindung enthält, die Aminogruppen enthält.

9. Die Verwendung eines Reduktionsmittels, wobei das Reduktionsmittel ein Borhydrid ist, zur Reduktion des Gehaltes an Aldehyden und Ketonen in Glycerin oder zur Erhöhung der Lagerstabilität von Glycerin.

## Claims

1. A process for the production of glycerol comprising
• providing a vegetable oil or fat,
• obtaining crude glycerol from the vegetable oil or fat and
• treating the crude glycerol with a reducing agent, the reducing agent being a borohydride, and the process additionally comprising
• removal of the boron in the form of a boron compound from the glycerol by distillation after treatment with the borohydride.

2. The process claimed in claim 1 additionally comprising
• distillation of the crude glycerol before it is treated with the reducing agent.

3. The process claimed in claim 2 additionally comprising
• the removal of soap from the crude glycerol before it is treated with the reducing agent and
• the treatment of the crude glycerol with an adsorbent, before it is treated with the reducing agent.

4. The process claimed in any of claims 1 to 3, the treatment of the crude glycerol with a reducing agent being carried out at a pH above 7 and this pH being adjusted by the addition of a base.

5. The process claimed in any of claims 1 to 4, the reducing agent being sodium borohydride and the sodium borohydride being used in a concentration of 0.005 to 0.05% by weight, based on the crude glycerol, and the treatment of the crude glycerol with the sodium borohydride being carried out at a temperature of 60 to 100°C.

6. The process claimed in any of claims 1 to 5, the crude glycerol being obtained from the vegetable oil or fat exclusively at temperatures below 100°C and exclusively under pressure below 2 bar (so-called low temperature/low pressure process).

7. A process for the production of a medicament, the medicament containing glycerol and at least one pharmaceutical active principle and the process comprising
a) the process claimed in any of claims 1 to 6 and
b) contacting of the glycerol with at least one pharmaceutical active principle.

8. The process for producing a medicament claimed in claim 7, the medicament containing a protein or the medicament containing a compound which contains amino groups.

9. The use of a reducing agent, the reducing agent being a borohydride, for reducing the content of aldehydes and ketones in glycerol or for increasing the storage stability of glycerol.

## Revendications

1. Procédé pour la production de glycérol, comprenant
• la fourniture d'une huile ou graisse végétale,
• l'obtention de glycérol brut à partir de l'huile ou de la graisse végétale et
• le traitement du glycérol brut par un réducteur,
le réducteur étant l'hydrure de bore et le procédé comprenant en outre
• l'élimination du bore sous forme de composé de bore à partir du glycérol par distillation, après le traitement par l'hydrure de bore.

2. Procédé selon la revendication 1, comprenant en outre
• la distillation du glycérol brut, avant qu'il soit traité par le réducteur.

3. Procédé selon la revendication 2, comprenant en outre
• une séparation de savon à partir du glycérol brut, avant qu'il soit traité par le réducteur, et
• le traitement du glycérol brut par un adsorbant, avant qu'il soit traité par le réducteur.

4. Procédé selon l'une quelconque des revendications 1 à 3, dans lequel le traitement du glycérol brut par un réducteur est effectué à un pH supérieur à 7, et l'ajustement de ce pH s'effectuant par l'addition d'une base.

5. Procédé selon l'une quelconque des revendications 1 à 4, dans lequel le réducteur est le borohydrure de sodium et le borohydrure de sodium étant utilisé à une concentration de 0,005 à 0,05 % en poids, par rapport au glycérol brut, et dans lequel le traitement du glycérol brut par le borohydrure de sodium est effectué à une température de 60 à 100 °C.

6. Procédé selon l'une quelconque des revendications 1 à 5, dans lequel l'obtention du glycérol brut à partir de l'huile ou de la graisse végétale s'effectue exclusivement à des températures de moins de 100 °C et exclusivement sous des pressions de moins de 2 bars (procédé dit basse température-basse pression).

7. Procédé pour la fabrication d'un médicament, dans lequel le médicament comprend du glycérol et au moins une substance active pharmaceutique, et le procédé comprenant
a) le procédé selon l'une quelconque des revendications 1 à 6 et
b) la mise en contact du glycérol avec au moins une substance active pharmaceutique.

8. Procédé pour la fabrication d'un médicament selon la revendication 7, dans lequel le médicament contient une protéine, ou dans lequel le médicament contient un composé qui contient des groupes amino.

9. Utilisation d'un réducteur, le réducteur étant le borohydrure de sodium, pour la réduction de la teneur en aldéhydes et cétones du glycérol ou pour l'augmentation de la stabilité au stockage du glycérol.
